(19) Europäisches Patentamt
European Patent Office
Office européen des brevets

(11) Veröffentlichungsnummer : **0 034 666**
**B1**

(12) # EUROPÄISCHE PATENTSCHRIFT

(45) Veröffentlichungstag der Patentschrift : **24.08.83**

(51) Int. Cl.$^3$ : **C 07 D207/27**

(21) Anmeldenummer : **80200288.1**

(22) Anmeldetag : **28.03.80**

(54) **Verfahren zur Herstellung von substituierten Essigsäurehydraziden.**

(30) Priorität : **23.02.80 DE 3006807**

(43) Veröffentlichungstag der Anmeldung : **02.09.81 Patentblatt 81/35**

(45) Bekanntmachung des Hinweises auf die Patenterteilung : **24.08.83 Patentblatt 83/34**

(84) Benannte Vertragsstaaten : **AT BE CH DE FR GB IT LU NL SE**

(56) Entgegenhaltungen :
**DE A 2 440 633**
**DE A 2 745 907**

**Chemical Abstracts, Band 92, Nr. 21, 26. Mai 1980 COLUMBUS, OHIO, USA Seite 637, Spalte 1, Abstract Nr. 180999p**

(73) Patentinhaber : **Rütgerswerke Aktiengesellschaft**
**Mainzer Landstrasse 217**
**D-6000 Frankfurt a.Main 1 (DE)**

(72) Erfinder : **Orth, Winfried, Dr.**
**Am Schachtelgraben 28**
**D-6733 Hassloch/Pfalz (DE)**
Erfinder : **Lange, Fritz Walter**
**Koenigswieserstrasse 26**
**D-8035 Gauting (DE)**
Erfinder : **Fickert, Werner, Dr.**
**Stockacher Strasse 14**
**D-6800 Mannheim-Seckenheim (DE)**

**EP 0 034 666 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schrifflich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von substituierten Essigsäurehydraziden

Die Erfindung betrifft ein Verfahren zur Herstellung von substituierten Essigsäurehydraziden der allgemeinen Formel I

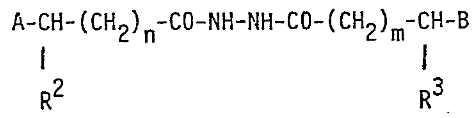
(I)

worin A und B gleich oder verschieden sind und einen Rest der Formel

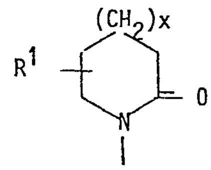

bedeuten, worin $R^1$ Wasserstoff oder eine Alkylgruppe mit 1 bis 4 C-Atomen ist, x den Wert 0 oder 1 hat, und worin $R^2$ und $R^3$ gleich oder verschieden sind und Wasserstoff oder eine Alkylgruppe mit 1 bis 4 C-Atomen bedeuten, n und m gleich oder verschieden sind und 0, 1, 2 oder 3 bedeuten. Die Alkylgruppen können geradkettig oder verzweigt sein.

N,N'-Bisacylhydrazide können durch Umsetzung eines entsprechenden Esters mit einem entsprechenden Säurehydrazid hergestellt werden. Auf diese Weise werden nach der DT-OS 2 440 633 oder der deutschen Patentanmeldung P 27 45 907 der F. W. Lange GmbH & Co. KG Verbindungen der Formel I hergestellt, indem man eine Verbindung der Formel II

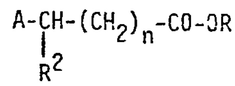
(II)

worin R eine Alkylgruppe mit 1-4 Kohlenstoffatomen und vorzugsweise eine Methylgruppe bedeutet, mit einem Hydrazidderivat der Formel III

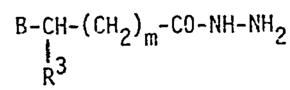
(III)

umsetzt. Selbst bei Verwendung des bevorzugt eingesetzten Methylesters (R— $CH_3$) verläuft die Reaktion aber mit einer wirtschaftlich wenig befriedigenden Ausbeute von maximal ca. 50-55 %.

Aufgabe der Erfindung war es daher, ein Verfahren zur Herstellung von N,N'-Bisacylhydraziden zu finden, das in einfacher Weise mit guten Ausbeuten durchführbar ist, und die Verbindungen I in der gearde für den Einsatz als Pharmazeutika notwendigen hohen Reinheit liefert.

Diese Aufgabe wird durch die vorliegende Erfindung gelöst. Die Erfindung ist dadurch gekennzeichnet, daß man die Umsetzung der Verbindungen der allgemeinen Formel II mit einem Hydrazid der Formel III in Gegenwart einer Verbindung R'OMe durchführt, worin R' ein Wasserstoffatom oder eine Alkylgruppe mit 1 bis 7, vorzugsweise 1 bis 4, C-Atomen darstellt und Me ein Alkalimetall, insbesondere Kalium oder Natrium, bedeutet. Eine Alkylgruppe R oder R' kann geradkettig oder verzweigt sein.

Nach diesem Verfahren lassen sich überraschenderweise die Verbindungen der Formel I mit einer wesentlich höheren Ausbeute, die durchschnittlich 70-85 % der Theorie beträgt, herstellen.

Als Katalysator kann auch ein Gemisch zweier oder mehrerer der Verbindungen R'OMe verwendet werden.

Die Verbindungen der Formel II und III werden im allgemeinen im molaren Verhältnis 1 : 1, oder auch mit einem geringen Überschuß (ca. 0,1-0,4 Mol) an II oder III umgesetzt.

Die Menge an zugesetzter Verbindung R'OMe ist nicht kritisch und kann z. B. von 0,01 bis 50 Mol-%, bezogen auf die Ausgangssubstanzen II oder III, betragen. Vorzugsweise werden Mengen von 0,01-1,0 und insbesondere in Mengen von 0,05 bis 0,2 Mol-% zugesetzt.

Die Umsetzung kann ohne oder insbesondere in Gegenwart eines inerten organischen Lösungsmittels durchgeführt werden. Solche Lösungsmittel sind z. B. niedere Alkanole, wie z. B. Methanol oder Isopropanol, oder vorzugsweise aromatische Kohlenwasserstoffe, wie z. B. Toluol und insbesondere Xylol, oder auch Gemische davon.

Die Umsetzung wird im allgemeinen bei erhöhter Temperatur, vorzugsweise zwischen 100 und 180 °C, und insbesondere zwischen 120 und 140 °C durchgeführt, wobei die Wahl der Temperatur auch

vom verwendeten Katalysator abhängig ist. In bestimmten Fällen kann es auch zweckmäßig sein, das Reaktionsgemisch anfänglich zu kühlen. Die Reaktion kann unter vermindertem Druck, unter erhöhtem Druck im geschlossenen Gefäß, oder insbesondere bei Normaldruck durchgeführt werden. Das Arbeiten unter einer inerten Gasatmosphäre, z. B. mit Stickstoff, kann vorteilhaft sein.

Verbindungen der allgemeinen Formel I besitzen wertvolle pharmakologische Eigenschaften und können z. B. als Psychopharmaka eingesetzt werden. Das erfindungsgemäße Verfahren eignet sich insbesondere zur Herstellung des pharmakologisch hochwirksamen N,N'-Bis-(pyrrolidinon-(2)-1-essigsäure)-hydrazids. Die Verbindungen der Formel III können aus den Verbindungen der Formel II und Hydrazin nach dem Verfahren der DT-OS 2 440 633 hergestellt werden.

Bei symmetrischen Bishydraziden kann das erfindungsgemäße Verfahren auch so ausgeführt werden, daß man die Verbindungen der Formel III aus dem bei ihrer Herstellung aus II und Hydrazin erhaltenen Reaktionsgemisch nicht isoliert, sondern zum Reaktionsgemisch direkt die für die weitere Umsetzung erforderliche Menge an Verbindung II zufügt; die Verbindung II kann auch schon vor der Zugabe von Hydrazin in doppelter Menge vorhanden sein. Bei dieser Verfahrensvariante kann die Verbindung R'OMe schon der anfänglichen Mischung aus II und Hydrazin zugesetzt werden, vorzugsweise und bei Verwendung von Hydrazinhydrat wird sie aber dem Reaktionsgemisch erst nach der Umsetzung von II mit Hydrazin zugefügt.

Die nachfolgenden Beispiele erläutern das erfindungsgemäße Verfahren näher, ohne es dadurch einzuschränken.

## Beispiel 1

N,N-Bis-(pyrrolidinon-(2)-1-essigsäure)-hydrazid

Eine Mischung aus 157 g (1 Mol) Pyrrolidinon-(2)-1-essigsäurehydrazid, 157 g (1 Mol) Pyrrolidinon-(2)-essigsäuremethylester und 500 ml Xylol wird auf 100 °C erwärmt und dann unter Rühren eine Lösung von 8,1 g (0,15 Mol) Natriummethylat in 100 ml Methanol so zugetropft, wie das Methanol über eine kleine Kolonne wieder abzudestillieren vermag.

Anschließend wird der Kolbeninhalt 24 Stunden unter Rühren auf 130 ± 1 °C erhitzt, wobei das infolge der fortschreitenden Reaktion freiwerdende Methanol über eine kleine Kolonne derart abdestilliert wird, daß die Sumpftemperatur konstant bleibt.

Danach wird die Mischung bei 90 °C mit 150 ml Isopropanol versetzt und unter Rühren 1 Stunde unter Rückfluß erhitzt, danach auf Raumtemperatur abgekühlt, das Kristallisat abgesaugt, der Filterkuchen mit 3 × 150 ml Isopropanol nachgewaschen und im Vakuum bei 60-70 °C getrocknet.

Schmelzpunkt: 201-3 °C

Ausbeute: 242-245,6 g (86-7 % d. Th.).

## Beispiele 2-14

| Nr. | Kondensationsmittel | (gelöst in .....) | Einsatz Mol-% | Ausbeute % d. Th. |
|---|---|---|---|---|
| 2 | Natriumhydroxid | (Methanol) | 0,15 | 76,3 |
| 3 | Kaliumhydroxid | (Methanol) | 0,15 | 78,7 |
| 4 | Kaliummethylat | (Methanol) | 0,10 | 85,3 |
| 5 | Kaliummethylat | (Methanol) | 0,15 | 87,2 |
| 6 | Natriumäthylat | (Äthanol) | 0,12 | 83,8 |
| 7 | Natriumäthylat | (ohne) | 0,15 | 82,1 |
| 8 | Kaliumäthylat | (Äthanol) | 0,1 | 83,5 |
| 9 | Natriumisopropylat | (Isopropanol) | 0,1 | 82,8 |
| 10 | Lithiummethylat | (Methanol) | 0,15 | 77,1 |
| 11 | Kalium-sec.-butylat | (sec.-Butanol) | 0,15 | 83,1 |
| 12 | Natrium-tert.-butylat | (ohne) | 0,1 | 81,1 |
| 13 | Kalium-tert.-butylat | (ohne) | 0,08 | 82,2 |
| 14 | Natriumbutylat | (ohne) | 0,15 | 84,3 |

Beispiel 15

N,N-Bis-(pyrrolidinon-(2)-1-essigsäure)-hydrazid

Eine Mischung aus 157 g (1 Mol) Pyrrolidinon-(2)-1-essigsäurehydrazid, 171 g (1 Mol) Pyrrolidinon-(2)-1-essigsäureäthylester und 500 ml Xylol wird auf 100 °C erwärmt und dann unter Rühren einer Lösung von 12,6 g (0,15 Mol) Kaliumäthylat in 150 ml Äthanol so zugetropft, wie das Äthanol über eine kleine Kolonne wieder abdestillieren vermag.

Anschließend wird der Kolbeninhalt 24 Stunden unter Rühren auf 130 ± 1 °C erhitzt, wobei das infolge der fortschreitenden Reaktion freiwerdende Äthanol über eine kleine Kolonne derart abdestilliert wird, daß die Sumpftemperatur konstant bleibt.

Danach wird die Mischung bei 90 °C mit 150 ml Isopropanol versetzt und unter Rühren 1 Stunde unter Rückfluß erhitzt, danach auf Raumtemperatur abgekühlt, das Kristallisat abgesaugt, der Filterkuchen mit 3 × 150 ml Isopropanol nachgewaschen und im Vakuum bei 60-70 °C getrocknet.

Schmelzpunkt : 202-3 °C

Ausbeute : 233-241 (82,5-85,3 % d. Th.)

Zur Entfernung eines Ascheanteils wird das Material aus Methanol umkristallisiert.

Beispiel 16

N,N'-Bis-(5-methyl-pyrrolidinon-(2)-1-essigsäure)-hydrazid

Zu einer Mischung aus 171 g (1 Mol) 5-Methyl-pyrrolidinon-(2)-1-essigsäurehydrazid, 171 g (1 Mol) 5-Methyl-pyrrolidinon-(2)-1-essigsäuremethylester und 300 ml Xylol werden unter Rühren portionsweise 16,8 g (0,15 Mol) Kalium-tert.-butylat gegeben und anschließend auf 130 ± 1 °C erhitzt, wobei die infolge der fortschreitenden Reaktion freiwerdenden niedrigsiedenden Alkohole (Methanol und tert.-Butanol) über eine kleine Kolonne derart abdestilliert werden, daß die Sumpftemperatur konstant bleibt.

Danach wird die Mischung bei 90 °C mit 150 ml Isopropanol versetzt und unter Rühren 1 Stunde unter Rückfluß erhitzt, danach auf Raumtemperatur abgekühlt, das Kristallisat abgesaugt, der Filterkuchen mit 3 × 150 ml Isopropanol nachgewaschen und im Vakuum bei 60-70 °C getrocknet.

Schmelzpunkt : 181 °C

Ausbeute : 245-258 g (79-83 % d. Th.).

**Ansprüche**

1. Verfahren zur Herstellung von substituierten Essigsäurehydraziden der allgemeinen Formel I

$$A-\underset{R^2}{\underset{|}{CH}}-(CH_2)_n-CO-NH-NH-CO-(CH_2)_m-\underset{R^3}{\underset{|}{CH}}-B \qquad (I)$$

worin A und B gleich oder verschieden sind und einen Rest der Formel

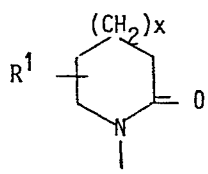

bedeuten, worin $R^1$ Wasserstoff oder eine Alkylgruppe mit 1 bis 4 C-Atomen ist, x den Wert 0 oder 1 hat, und worin $R^2$ und $R^3$ gleich oder verschieden sind und Wasserstoff oder eine Alkylgruppe mit 1 bis 4 C-Atomen bedeuten, n und m gleich oder verschieden sind und 0, 1, 2 oder 3 bedeuten, durch Umsetzung einer Verbindung der Formel II

$$A-\underset{R^2}{\underset{|}{CH}}-(CH_2)_n-CO-OR \qquad (II)$$

mit einem Hydrazidderivat der Formel III

$$B-\underset{R^3}{\underset{|}{CH}}-(CH_2)_m-CO-NH-NH_2 \qquad (III)$$

worin A, B, $R^1$, $R^2$, $R^3$, x, m und n die oben angegebene Bedeutung haben und R eine Alkylgruppe mit 1-4 C-Atomen ist, dadurch gekennzeichnet, daß man die Umsetzung in Gegenwart einer Verbindung R'OMe durchführt, worin R' ein Wasserstoffatom oder eine Alkylgruppe mit 1 bis 7 C-Atomen darstellt und Me ein Alkalimetall bedeutet.

2. Verfahren nach Anspruch 1, dadurch gekennzeichnet, daß man die Umsetzung in Gegenwart einer Verbindung R'OMe durchführt, worin R' eine Alkylgruppe mit 1 bis 4 C-Atomen darstellt.

3. Verfahren nach Anspruch 1 und 2, dadurch gekennzeichnet, daß man die Umsetzung in Gegenwart einer Verbindung R'OMe durchführt, worin Me Kalium oder Natrium bedeutet.

4. Verfahren nach einem der Ansprüche 1 bis 3, dadurch gekennzeichnet, daß man die Verbindung R'OMe in einer Menge von 0,01-1,0 Mol-%, bezogen auf die Verbindungen II oder III, zusetzt.

5. Verfahren nach einem der Ansprüche 1 bis 4, dadurch gekennzeichnet, daß man die Verbindung R'MOe in einer Menge von 0,05 bis 0,2 Mol-%, bezogen auf die Verbindungen II oder III, zusetzt.

6. Verfahren nach einem der Ansprüche 1 bis 5, dadurch gekennzeichnet, daß man bei einer Temperatur von 100 bis 180 °C arbeitet.

7. Verfahren nach einem der Ansprüche 1 bis 6, dadurch gekennzeichnet, daß man bei einer Temperatur von 120 bis 140 °C arbeitet.

8. Verfahren nach einem der Ansprüche 1 bis 7, dadurch gekennzeichnet, daß man N,N'-Bis(pyrrolidinon-(2)-1-essigsäure)-hydrazid herstellt.

9. Verfahren nach einem der Ansprüche 1 bis 8, dadurch gekennzeichnet, daß man bei der Herstellung symmetrischer Bishydrazide der Formel I die Verbindungen der Formel III aus dem bei ihrer Herstellung aus II und Hydrazin erhaltenen Reaktionsgemisch nicht isoliert, sondern zum Rekationsgemisch direkt die für die weitere Umsetzung erforderliche Menge an Verbindung II zufügt.

## Claims

1. A process for the preparation of substituted acylhydrazides of the general formula

$$A-\underset{\underset{R^2}{|}}{CH}-(CH_2)_n-CO-NH-NH-CO-(CH_2)_m-\underset{\underset{R^3}{|}}{CH}-B \qquad (I)$$

wherein A and B are identical or different and mean a radical of the formula

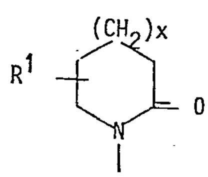

wherein $R^1$ is selected from the group consisting of hydrogen and alkyl of 1 to 4 carbon atoms, x is 0 or 1 and $R^2$ and $R^3$ are individually selected from the group consisting of hydrogen and alkyl of 1 to 4 carbon atoms and n and m are individually 0, 1, 2 or 3 by reacting a compound of the formula

$$A-\underset{\underset{R^2}{|}}{CH}-(CH_2)_n-CO-OR \qquad (II)$$

with a hydrazide of the formula

$$B-\underset{\underset{R^3}{|}}{CH}-(CH_2)_m-CO-NH-NH_2 \qquad (III)$$

the improvement comprising effecting the reaction in the presence of at least one catalyst of the formula R'-MOe wherein R' is selected from the group consisting of hydrogen and alkyl of 1 to 7 carbon atoms and Me is an alkali metal.

2. The process of claim 1 wherein the reaction is effected in the presence of a catalyst R'-MOe wherein R' is an alkyl radical of 1 to 4 carbon atoms.

3. The process of claims 1 and 2 wherein the reaction is effected in the presence of a catalyst R'-OMe wherein Me is potassium or sodium.

4. The process of claims 1 to 3 wherein the amount of catalyst R'-OMe is 0.01 to 1.0 mole-percent based on the amount of the compound of formula II or III.

5. The process of claims 1 to 4 wherein the amount of catalyst R'-OMe is 0,05 to 0,2 mole-percent based on the amount of the compound of formula II or III.

6. The process of claims 1 to 5 wherein the reaction is effected at 100 to 180 °C.

7. The process of claims 1 to 6 wherein the reaction is effected at 120 to 140 °C.

8. The process of claims 1 to 7 wherein N,N'-Bis(pyrrolidinone-(2)-1 acetic acid)-hydrozide is prepared.

9. The process of claims 1 to 8 wherein the symetrical bishydrazide of formula III is formed by reacting a compound of formula II with hydrazine and the latter is reacted without isolation with another mole of the compound of formula II.

**Revendications**

1. Procédé de préparation d'hydrazides d'acides acétiques substitués qui répondent à la formule générale I :

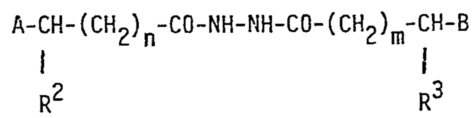

(I)

dans laquelle A et B sont identiques ou différents et représentent chacun un radical de formule :

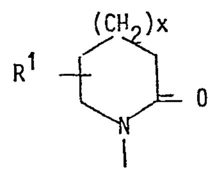

(où $R^1$ représente l'hydrogène ou un radical alkyle contenant de 1 à 4 atomes de carbone et x est égal à 0 ou à 1), $R^2$ et $R^3$ sont identiques ou différents et représentent chacun l'hydrogène ou un radical alkyle contenant de 1 à 4 atomes de carbone, et n et m sont égaux chacun, indépendamment l'un de l'autre, à 0, à 1, à 2 ou à 3, par réaction d'un composé de formule II :

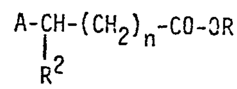

(II)

avec un hydrazide de formule III :

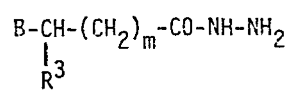

(III)

formules dans lesquelles A, B, $R^1$, $R^2$, $R^3$, x, m et n ont les significations précédemment données et R représente un radical alkyle contenant de 1 à 4 atomes de carbone, procédé caractérisé en ce qu'on effectue la réaction en présence d'un composé R'OMe dans lequel R' désigne un atome d'hydrogène ou un radical alkyle contenant de 1 à 7 atomes de carbone et Me représente un métal alcalin.

2. Procédé selon la revendication 1, caractérisé en ce qu'on effectue la réaction en présence d'un composé R'OMe dans lequel R' représente un radical alkyle contenant de 1 à 4 atomes de carbone.

3. Procédé selon l'une des revendications 1 et 2, caractérisé en ce qu'on effectue la réaction en présence d'un composé R'OMe dans lequel Me représente le potassium ou le sodium.

4. Procédé selon l'une quelconque des revendications 1 à 3, caractérisé en ce qu'on ajoute le composé R'OMe en une quantité de 0,01 à 1,0 % en moles par rapport aux composés II ou III.

5. Procédé selon l'une quelconque des revendications 1 à 4, caractérisé en ce qu'on ajoute le composé R'OMe en une quantité de 0,05 à 0,2 % en moles par rapport aux composés II ou III.

6. Procédé selon l'une quelconque des revendications 1 à 5, caractérisé en ce qu'on opère à une température de 100 à 180 °C.

7. Procédé selon l'une quelconque des revendications 1 à 6, caractérisé en ce qu'on opère à une température de 120 à 140 °C.

8. Procédé selon l'une quelconque des revendications 1 à 7, caractérisé en ce qu'on prépare la N,N'-bis-[(oxo-2 pyrrolidinyl-1)-acétyl]-hydrazine.

9. Procédé selon l'une quelconque des revendications 1 à 8, caractérisé en ce que, dans la préparation de bishydrazides symétriques répondant à la formule I, on n'isole pas les composés de formule III du mélange réactionnel obtenu lors de leur préparation à partir de II et de l'hydrazine, mais on ajoute directement au mélange réactionnel la quantité de composé II nécessaire pour la réaction ultérieure.